# EUROPEAN PATENT SPECIFICATION

(11) **EP 3 177 294 B1**
(45) Date of publication and mention of the grant of the patent: **23.03.2022**
(21) Application number: 14899355.3
(22) Date of filing: 19.12.2014
(51) Int. Cl.: A61K 31/519, A61K 31/522, A61K 35/17, A61K 45/06, A61P 35/00, A61P 35/02, A61P 43/00

(54) **COMPOSITIONS COMPRISING A PNP INHIBITOR FOR USE IN THE TREATMENT OF THE RELAPSE OF MALIGNANCY FOLLOWING HEMATOPOIETIC STEM CELL TRANSPLANT**
ZUSAMMENSETZUNGEN ENTHALTEND EIN PNP-INHIBITOR ZUR BEHANDLUNG DES RÜCKFALLS VON MALIGNITÄT NACH EINER HÄMATOPOETISCHEN STAMMZELLTRANSPLANTATION
COMPOSITIONS CONTENTANT UN INHIBITEUR DE PNP DESTINÉES AU TRAITEMENT DE RÉCIDIVE DE TUMEUR MALIGNE APRÈS TRANSPLANTATION DE CELLULES SOUCHES HÉMATOPOÏÉTIQUES

(30) Priority: 07.08.2014 US 201462034323 P
(43) Date of publication of application: 14.06.2017
(73) Proprietor: Laevoroc Immunology AG, 6331 Hünenberg (CH)
(72) Inventor: BANTIA, Shanta, Birmingham, Alabama 35242 (US)
(74) Representative: Stratagem IPM Limited
(86) International application number: PCT/US2014/071508
(87) International publication number: WO 2016/022166

(56) References cited:
- WO-A1-2014/205194
- WO-A2-2004/002425
- WO-A2-2006/072093
- WO-A2-2014/085437
- US-A1- 2003 114 466
- US-A1- 2011 038 858
- US-A1- 2011 038 858
- US-A1- 2014 154 225
- G. B. EVANS ET AL: "Transition State Analogue Inhibitors of N-Ribosyltransferases: New Drugs by Targeting Nucleoside Processing Enzymes.", NUCLEIC ACIDS SYMPOSIUM SERIES, vol. 51, no. 1, 1 November 2007 (2007-11-01), pages 63-64, XP055425641, GB ISSN: 0261-3166, DOI: 10.1093/nass/nrm032
- Mairéad Níchonghaile: "Graft Versus Tumour Effect" In: "The European Blood and Marrow Transplantation Textbook for Nurses", 22 November 2017 (2017-11-22), Springer International Publishing, Cham, XP055744728, ISBN: 978-3-319-50026-3 pages 253-258, DOI: 10.1007/978-3-319-50026-3_12,

## Description

### CROSS-REFERENCE TO RELATED APPLICATIONS

The present application claims priority to US Provisional Application 62/034,323 filed 07 August 2014.

### Technical Field

The invention relates to pharmaceutical compositions for use in methods for enhancing Graft versus Malignancy (GvsM) effects in patients with relapse of malignancy after hematopoietic stem cell transplantation.

### Background

Allogeneic Hematopoietic stem cell transplantation (HSCT) is curative in some patients with high-risk leukemia but is not always successful. Despite attempts in enhancing the efficacy of conditioning regimens, disease relapse still commonly occurs. Several approaches have been used to treat leukemia relapse following HSCT, including discontinuation of immunosuppression, re-induction of chemotherapy, or repeat transplantation, with only limited success. Donor lymphocyte infusion (DLI) is another approach that is currently being used.

DLI is a form of adoptive immunotherapy to induce GvsM effects. The efficacy of this approach is dependent upon the type of disease and the dose of infused CD3⁺ lymphocytes. Clinical success has primarily been limited to those with CML and indolent lymphoma. The response is generally limited in florid relapse of acute myeloid leukemia (AML) or acute lymphoblastic leukemia (ALL), and patients are at risk of severe graft-versus-host disease (GvHD) or marrow aplasia. Inadequate stimulation of T cells may be one mechanism underlying failure of adoptive immunotherapy after HSCT. Hence drugs that can induce GvsM effects by enhancing the stimulation of donor T-cells and other immune cells so as to reverse or inhibit relapse or progression of malignancy would be highly desirable for patients with relapse of malignancy after HSCT.

WO 2004/002425 discloses donor lymphocyte infusion in the treatment of patients having multiple myeloma who have relapsed following bone marrow transplantation.

WO 2014/085437 discloses MIL (marrow infiltrating lymphocyte) infusion in the treatment of patients having multiple myeloma who have relapsed following bone marrow transplantation.

WO 2006/072093 discloses a method of treating the relapse of cancer in an animal following a bone marrow or stem cell transplant procedure by orally administering a pharmaceutically-effective amount of an immunosuppressive agent.

Development of immune-potentiating agents is one of the strategies that could potentially be used to enhance GvsM effect (Bashey et.al in Blood (2009) 7:1581; Bouchlaka et. al., in Immunotherapy (2010) 2(3): 399). An adjuvant is an agent administered to potentiate the immune response to an antigen and/or modulate it towards a desired immune response. An endogenous adjuvant is a compound or molecule naturally occurring within the cell or tissue that likewise enhances an immune response by stimulating innate immunity, thus possessing the capacity to potentiate an effect of some triggering event or agent. Endogenous adjuvants play a central role in alerting the immune system to potential danger, and promote response to infection, transplantation, tumor, and autoimmunity.

Purine nucleoside phosphorylase (PNP)-deficient patients demonstrate significantly high levels of plasma purine nucleoside guanosine compared to normal healthy subjects (Markert in Immunodeficiency Review (1991) 3:45-81). A major source of purine nucleoside pools comes from the breakdown of RNA and DNA during normal cell turnover, cellular injury or cell death due to infection. Normally guanosine is present at very low to undetectable levels in the plasma because PNP is an extremely efficient catalyst and rapidly breaks down guanosine to guanine and ribose 1-phosphate (Markert in Immunodeficiency Review (1991) 3:45-81). In the presence of PNP inhibitor or due to a PNP deficiency, purine nucleoside guanosine is elevated in the plasma.

Toll-like receptors (TLRs) are an established family of pathogen recognition receptors (PRRs) that initiate the innate immune response. In addition to TLRs there are other PRR's like retinoic acid inducible gene I (RIGI) like receptors (RLR), nucleotide binding oligomerization domain (NOD)-like receptors (NLR) as well as c-type lectin receptors (CLR). Stimulation of TLRs and PRRs directly or indirectly causes the release of multiple cytokines including type 1 and type 2 interferons, the induction of pathways and enzymes that destroy intracellular pathogens, the activation of a variety of cellular responses, and the priming of the adaptive response by activating immature dendritic cells and inducing their differentiation into professional antigen-presenting cells. At least eleven different TLR genes have been identified in humans.

TLRs recognize highly conserved structural motifs known as pathogen-associated microbial patterns (PAMPs) which are exclusively expressed by microbial pathogens or danger-associated molecular patterns (DAMPs) that are endogenous molecules released from necrotic or dying cells. Stimulation of TLRs by corresponding PAMPs or DAMPs initiates signaling cascades leading to activation of the immune system.

Clearly it would be beneficial to provide methods for treating diseases which exploit the natural endogenous adjuvant response. Controlling levels of metabolites (for example, DAMPs) that can act as TLR agonists, provides a novel means to stimulate donor T-cells and other immune cells, and thereby to enhance GvsM effects. Further, identification of endogenous adjuvants triggered in response to certain pathogens provides novel exogenous adjuvants which may be administered to stimulate donor T-cells to enhance GvsM effects in patients with relapse of malignancy post HSCT.

### Summary

The invention is defined by the claims. Any subject-matter falling outside the scope of the claims is provided for information purposes only.

Any references in the description to methods of treatment, diagnosis or surgery refer to the compounds, pharmaceutical compositions and medicaments of the present invention for use in a method for treatment, diagnosis or surgery of the human or animal body by therapy.

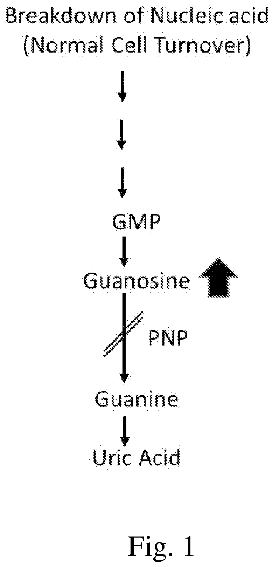

Contrary to expectations based on the immuno-compromised clinical phenotype of the PNP-deficient patient, the present disclosure describes that PNP inhibitors (PNPi) can act as immune-potentiators with a novel mechanism of action. Based on the role of PNP in purine catabolism, the present invention hypothesizes that inhibition of PNP leads to elevation of plasma guanosine (Fig 1) which can act as endogenous adjuvant by activating TLRs. Activation of TLRs results in innate immune activation which can then regulate adaptive immune system. In essence PNP inhibitors could act as immune potentiator by indirectly activating TLRs.

The present disclosure describes compositions and methods for inhibiting PNP and for effectuating an increase in guanosine levels in a subject. This endogenous substance behaves as an endogenous adjuvant and can act as an immune-enhancer by activating TLRs and stimulating donor T-cells in the presence of tumor antigens. This translates into increased GvsM effects in patients with relapse of malignancy after hematopoietic stem cell transplantation and reversal or prevention of relapse or progression of malignancy. Such compositions and methods were not previously appreciated in the art.

The present disclosure further describes compositions, kits and methods useful for inhibiting PNP in combination with guanosine which stimulate donor T-cells to enhance GvsM effects in patients with relapse of malignancy after hematopoietic stem cell transplantation, resulting in reversal or prevention of relapse and progression of malignancy. Such compositions and methods were not previously appreciated in the art.

Aspects of the present invention are expanded and clarified by reference to the Detailed Description set forth below.

### Brief Description of the Drawings

### BRIEF DESCRIPTION OF THE DRAWINGS

**Figure 1****.** Depicts a schematic illustration of the relationship between PNP inhibition and levels of guanosine.
**Figure 2****.** Illustrates the activity of NTR001, inosine and guanosine as single agents, and in combination, on seven different human TLRs (TLR2, 3, 4, 5, 7, 8 and 9) as a potential agonist.
**Figure 3****.** Data showing serum tetanus toxoid antibody titers on day 38 in vehicle- and PNP inhibitors NTR001- and NTR002-treated mice groups in the tetanus toxoid mouse model.
**Figure 4****.** Depicts serum interferon-g levels on day 30 in vehicle and PNP inhibitors NTR001 and NTR002 treated mice groups in the tetanus toxoid mouse model.
**Figure 5****.** Demonstrates effect of PNP inhibitor NTR001 and chemotherapeutic agent cyclophosphamide on tumor volume in the mouse melanoma model.
**Figure 6****.** Demonstrations effect of PNP inhibitor NTR001 and chemotherapeutic agent cyclophosphamide on survival in the mouse melanoma model.
**Figure 7****.** Demonstrates effect of PNP inhibitor NTR001 on weight loss in the mouse model of *L. Monocytogenes* infection.
**Figure 8****.** Demonstrates effect of PNP inhibitor NTR001 on survival in the mouse model of *L. Monocytogenes* infection.
**Figure 9****.** Demonstrates effect of PNP inhibitor NTR002 on weight loss in the mouse model of *L. Monocytogenes* infection.
**Figure 10****.** Demonstrates effect of PNP inhibitor NTR002 on survival in the mouse model of *L. Monocytogenes* infection.
**Fig. 11****.** Demonstrates effect of PNP inhibitor NTR001 and CTLA4-Ab on tumor volume in the mouse melanoma model.
**Fig. 12****.** Demonstrates effect of PNP inhibitor NTR001 and CTLA4-Ab on survival in the mouse melanoma model.

### Detailed Description

TLRs play a critical role in the early innate immune response to, invading pathogens by sensing microorganism, and are also involved in sensing endogenous danger signals. TLRs recognize highly conserved structural motifs known as pathogen-associated microbial patterns (PAMPs), which are exclusively expressed by microbial pathogens, or danger-associated molecular patterns (DAMPs) that are endogenous molecules released from necrotic or dying cells. Stimulation of TLRs by the corresponding PAMPs or DAMPs initiates signaling cascades leading to the activation of transcription factors, such as AP-1, NF-KB and interferon regulatory factors (IRFs). Signaling by TLRs result in a variety of cellular responses including the production of interferons (IFNs), pro-inflammatory cytokines and effector cytokines that direct the adaptive immune response.

PNP (sometimes referred to as PNPase) deficiency is known to result in an increase in levels of guanosine which is the substrate of the enzyme. Guanosine can activate the immune system, more specifically donor T-cells, through TLRs and/or other PRRs in the presence of an appropriate antigen like tumor cells translating into enhanced GvsM effects in patients, with relapse of malignancy after hematopoietic stem cell transplantation, to reverse or prevent relapse or progression of malignancy.

Fig. 1 is a schematic presentation of the role of PNP in purine metabolism which illustrates the relationship between PNP inhibition and increases in guanosine levels.

PNP-deficient patients demonstrate high levels of plasma purine nucleoside guanosine. A major source of purine nucleoside pools comes from the breakdown of RNA and DNA during cellular injury or cell death. Normally, the purine nucleoside, guanosine is present at very low or undetectable levels in the plasma because PNP rapidly breaks down guanosine to guanine and sugar 1-phosphate. In the presence of PNP inhibitor or PNP deficiency guanosine is elevated which could potentially act as endogenous adjuvant and activate the immune system.

The present invention discerns, therefore, that increases in the PNP substrate guanosine due to inhibition of PNP could act as danger signal (endogenous adjuvant) and stimulate the donor T-cells and other immune cells to enhance the GvsM effects in patients, with relapse of malignancy after hematopoietic stem cell transplantation, to reverse or prevent relapse or progression of malignancy.

Guanosine analogs like isatoribine (7-thia, 8-oxoguanosine), loxorabine (7-allyl, 8-oxo guanosine) have demonstrated immune-potentiating effects, in-vitro studies with some guanosine analogs have shown activation of immune cells, for example dendritic cells and natural killer cells to produce ifn-gamma which is mediated through Toll-Like Receptor 7 (TLR7).

As used herein, "guanosine" is interpreted to include pharmacologically functional equivalents such as guanosine monophosphate (GMP), a precursor of guanosine. Pro-drugs of guanosine are also contemplated as within the scope, may be readily developed. Suitable pro-drugs of guanosine and synthesis thereof are set forth in Ray, Adrian S. et al. "Novel Use of a Guanosine Prodrug Approach To Convert 2',3'-Didehydro-2',3'-Dideoxyguanosine into a Viable Antiviral Agent" Antimicrob Agents Chemother. 2002 Mar; 46(3): 887-891, Zhang, Youxi et al. "Current prodrug strategies for improving oral absorption of nucleoside analogues" Asian Journal of Pharmaceutical Sciences. 2014 Apr; 9(2): 65-74, and Bourdin, C. et al. "Synthesis and evaluation against hepatitis C virus of 7-deaza analogues of 2'-C-methyl-6-O-methyl guanosine nucleoside and L-Alanine ester phosphoramidates" Bioorg Med Chem Lett 2013 Apr 20;23(7) 2260-4.

According to specific embodiments, the pro-drug of guanosine is selected from 6-O-methyl guanosine, 6-cyclopropyl amino guanosine, and combinations thereof.

Through stimulation of innate immunity by activating TLR, isatoribine and other guanosine analogs appear to prevent or reverse otherwise lethal viral infections in various acute infection models in mice. The present investigators therefore posit that by inhibiting PNP, guanosine and other nucleoside levels are elevated and may activate an immune response through TLRs and other PRRs like the guanosine analogs, isatoribine and loxoribine, translating into beneficial GvsM effects in patients who relapse post HSCT.

Embodiments of the present invention therefore provide methods for treating cancer which recognize and exploit the natural endogenous adjuvant response. Controlling/modulating the levels of endogenous adjuvants provide a novel means to enhance immunogenicity of an appropriate antigen related to tumor cells stimulating donor T-cells and other immune cells to increase GvsM effects in patients, with relapse of malignancy after hematopoietic stem cell transplantation, to reverse or prevent relapse or progression of malignancy.

Aspects of the invention relate to affirmatively inhibiting PNP to effectuate elevation of plasma guanosine, in a subject, as is observed in PNP deficient patients (Fig. 1).

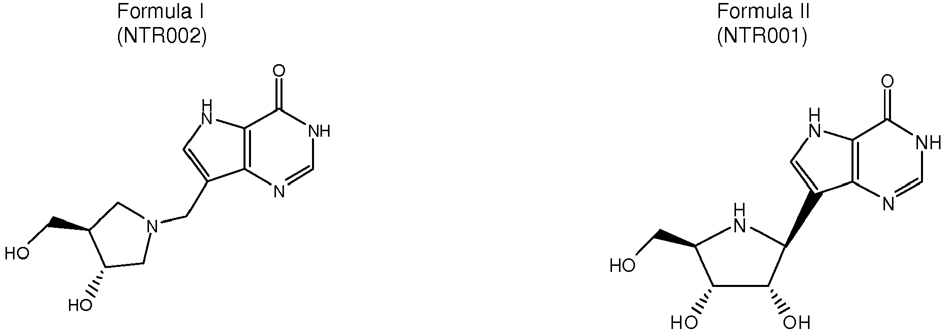

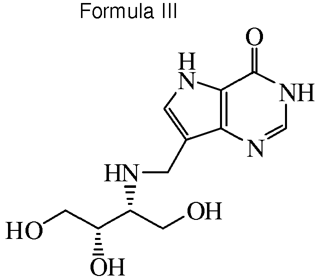

Compounds depicted structurally by Formula I (NTR002, also known as Ulodesine 1,5-dihydro-7-[[(3R,4R)-3-hydroxy-4-(hydroxymethyl)pyrrolidin-1-yl]methyl]-4H-pyrrolo[3,2-d]pyrimidin-4-one), Formula II (NTR001, also known as Forodesine 7-[(2*S*,3*S*,4*R*,5*R*)-3,4-dihydroxy-5-(hydroxymethyl)-2-pyrrolidinyl]-1,5-dihydro-4H-pyrrolo[3,2-d]pyrimidin-4-one) and Formula III have been shown to inhibit PNP. Further, structurally similar compounds known as transition state analogs have been studied as PNP inhibitors (Evans et al. in Organic Letters (2003) 5:3639; Taylor et al. in Journal of American Chemical Society (2007) 129:6984; Evans et al. in Journal of Medicinal Chemistry (2003) 46:5271; Castilho et al. in Bioorganic & Medicinal Chemistry (2006) 14:516; Schramm et al. in Journal of Biological Chemistry (2007) 282:28297; and Bantia et al. in International Immunopharmacology (2010) 784 and (2001) 1:1199-1210; Kicska et al. in Proceedings of National Academy of Sciences (2001) 98:4593-4598). Non-limiting examples of PNP inhibitors include those disclosed in U.S. Patent Numbers. 4,985,433; 4,985,434, 5,008,265; 5,008,270; 5,565,463 7,427,624, 5,721,240, 5,985,848, 7,390,890 and the continuation patents that are referenced therein, 7,109,331, 8,283,345, 8,173,662 and 7,553,839, and International Patent Number WO2008/030119 and 2. EP 2 395 005.

The term "PNP inhibitor" includes those compounds that inhibit PNP. Compositions having in-vitro inhibitory constant (Ki) values of less than about 5 x 10⁻⁶ M, typically less than about 1 X 10⁻⁷ M, and preferably less than 5 X 10⁻⁸ M are preferred for *in vivo* use.

In one embodiment, the present disclosure provides methods and compositions for inhibition of PNP and increase in the purine nucleoside, guanosine levels. In an alternate embodiment, the present disclosure provides for methods and compositions useful for enhancing GvsM effects in patients with relapse of malignancy post HSCT by a PNP inhibitor related to elevated guanosine, which can act as an endogenous adjuvant to stimulate the donor T-cells and other immune cells in subjects and enhance GvsM effects.

In another embodiment, the present disclosure further provides methods and compositions useful for enhancing GvsM effects in patients with relapse of malignancy after HSCT by administration of a combination of PNP inhibitors and a pharmaceutically effective amount of endogenous adjuvant, guanosine or a pro-drug of guanosine, or a precursor of guanosine (a substance from which guanosine is derived in the body; for example, guanosine mono phosphate (GMP)).

The present disclosure also provides articles of manufacture useful for increasing concentrations of guanosine in a subject. The present disclosure also provides articles of manufacture useful for enhancing GvsM effects in patients with relapse of malignancy post HSCT by increasing concentrations of guanosine in a subject. According to one specific embodiment, the article of manufacture comprises at least one reservoir containing a composition comprising one or more compounds structurally depicted by Formula I, Formula II, and Formula III, trivial variants thereof, PNP inhibitors listed in U.S. Patent Numbers 5,985,848, 6,066,722 and 7,553,839, and International Patent Number WO2008/030119, and an agent identified as endogenous adjuvant guanosine or a pro-drug of guanosine, or a precursor of guanosine. The articles of manufacture may be packaged with instructions relating to indications for various disorders that the compositions are capable of treating.

Compounds of Formulas I, II and III are 9-deazahypoxanthine derivatives. Compounds of Formula I, II, III and related compounds are described in U.S. Patent Numbers 5,985,848, 6,066,722 and 7,553,839 and International Patent Number WO2008/030119. In some embodiments of this disclosure, these compounds can exist as a pharmaceutically acceptable salt. In other embodiments these compounds can exist in a racemic equilibrium or as a specific tautomer. In yet other embodiments these compounds can exist as a solvate. In yet other embodiments these compounds can exist as a hydrate. In yet other embodiments these compounds can exist as a prodrug,

Furthermore, in the embodiments described above, the article of manufacture may contain a therapeutically effective amount of one or more compounds depicted by Formulas I, II, and III and related compounds such as those described in U.S. Patent Numbers 5,985,848, 6,066,722 and 7,553,839 and International Patent Number WO2008/030119, The one or more compounds may also be provided in combination with guanosine or a pro-drug of guanosine, or a precursor of guanosine.

In other specific embodiments the article of manufacture may further comprise, consist essentially of, or consist of, one or more additional active agents in combination with one or more of the compounds of Formula I, II, III and related compounds such as those described in U.S. Patent Numbers 5,985,848, 6,066,722 and 7,553,839, and in International Patent Number WO2008/030119. In more specific embodiments the combination may further include, guanosine or a pro-drug of guanosine, or a precursor of guanosine. Examples of active agents include but are not limited to analgesic agents, anti-inflammatory agents, anti-infective agents, anticancer agents, chemotherapeutic agents, agents that inhibit purine metabolism and other active agents know in the art.

With PNP inhibition, in addition to guanosine elevation, the other nucleosides and nucleotides that are elevated are inosine, deoxyinosine, deoxyguanosine, deoxyguanosine triphosphate (dGTP) and nicotinamide adenine dinucleotide (NAD). Those skilled in the art will recognize that, like guanosine, each of these nucleosides and nucleotides by itself or in combination can also act as endogenous adjuvant by activating TLRs and/or other PRR's like RLR, NLR, and CLR and thus stimulate the immune system. The invention disclosed is therefore not limited to the use of PNP inhibitor with guanosine but also the PNP inhibitor could be combined with any of the nucleosides and nucleotides by itself or in combination that are elevated with PNP inhibition to stimulate the immune system.

### Pharmaceutical Composition and Medicaments

Compounds may be administered as oral, parenteral, topical, or any other known method of administration in the literature and the formulations also may be prepared according to the requirements and the procedures reported in the literature. In specific embodiments, compositions comprising the compounds of the invention are formulated in delay-release or extended-release dose forms. For example, a compound comprising a PNP inhibitor may be formulated in a delay release form such that if administered together with another agent such that the PNP inhibitor will be released after the other agent is released in the subject.

### Dosages Administered

In one embodiment, useful dosages of the compound of the Formulas I, II and III, and related compounds described in U.S. Patent Numbers 5,985,848, 6,066,722 and 7,553,839, and International Patent Number WO2008/030119, can be determined by comparing their *in vitro* activity, and *in vivo* activity in animal models. Methods for the extrapolation of effective dosages in mice, and other animals, to humans are known to the art; for example, see U.S. Patent Number 4,938,949.

It is known that PNP inhibition in humans over long term at certain doses leads to decreases in various lymphocyte subsets (Gomes et al. in Blood ASH Annual Meeting Abstracts (2008) 112:Abstract 2583). Hence, long term treatment with high doses of PNP inhibitor may have immunosuppressive effects. Surprisingly, the present investigators discovered that PNP inhibitors exhibit an immune-potentiating effect in the presence of an antigen if doses are selected to avoid significant impact on lymphocytes.

The following Examples are set forth to illustrate certain aspects and features of the instant inventive subject matter and should not be construed as limiting the full scope as defined by the claims appended hereto. Example 1 described below demonstrate that guanosine, which is one of the nucleoside that is elevated when PNP is inhibited, activate TLR2 and TLR4. Activation of TLR2 and TLR4 results in immune potentiating effects as it leads to expression of transcription factors (like NF-kB ans IRF-3) resulting in expression of inflammatory cytokines and stimulation of T-cells and other immune cells.

Immune-potentiating agents demonstrate benefit in preclinical and clinical tests as anti-cancer agents, anti-infective agents and also can act as adjuvants in a vaccine. Examples 2, 3, 4 and 5 demonstrate the immune potentiating activity of PNP inhibitor in in-vivo mouse models of cancer, infection and vaccine. For purposes of interpreting this disclosure, Formulas I, II and III include trivial variants thereof, the term "trivial" being with respect to pharmaceutical efficacy. NTR001 is depicted structurally as Formula II, and NTR002 is depicted structurally as Formula I.

### EXAMPLE 1.

*Toll-Like Receptor (TLR) Ligand Screening: In-vitro Activity of the PNPi, guanosine and inosine on seven different human TLRs (TLR2, 3, 4, 5,* 7, 8 *and 9) as a potential agonist.*

TLRs play a critical role in the early innate immune response to invading pathogens by sensing microorganism and are involved in sensing endogenous danger signals. TLRs recognize highly conserved structural motifs known as pathogen-associated microbial patterns (PAMPs), which are exclusively expressed by microbial pathogens, or danger-associated molecular patterns (DAMPs) that are endogenous molecules released from necrotic or dying cells. Stimulation of TLRs by the corresponding PAMPs or DAMPs initiates signaling cascades leading to the activation of transcription factors, such as AP-1, NF-KB and interferon regulatory factors (IRFs). Signaling by TLRs result in a variety of cellular responses including the production of interferons (IFNs), pro-inflammatory cytokines and effector cytokines that direct the adaptive immune response. This Example investigates the potential TLR agonism of PNP inhibitors NTR001 (Foredesine set forth as Formula II), inosine and guanosine alone and in combination with respect to seven different human TLRs (TLR2, 3, 4, 5, 7, 8 and 9).

Method: TLR stimulation is tested by assessing NF-KB activation in HEK293 cells expressing a given TLR. The Secreted Embryonic Alkaline Phosphatase (SEAP) reporter is under the control of a promoter inducible by the transcription factor NF-KB. This reporter gene allows the monitoring of signaling through the TLR, based on the activation of NF-KB. The compounds are evaluated at one concentration and compared to control ligands. This step is performed in triplicate.

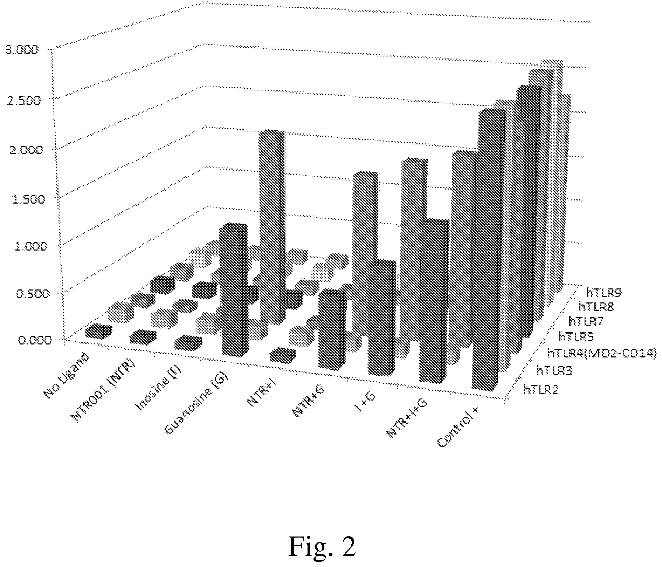

Results: Guanosine (100 uM) exhibits a significant stimulatory effect on human TLR2 and TLR4, alone or in combination with article NTR001 (10 uM) and/or Inosine (100 uM). NTR001, Inosine, and NTR001+Inosine do not exhibit a stimulatory effect on human TLR2, 3, 4, 5, 7, 8 or 9. (Fig. 2)

Conclusion: Guanosine is an agonist of TLR2 and TLR4 receptors. Activation of TLR2 and TLR4 results in immune activation and hence treatment with a combination guanosine and PNPi (to prevent breakdown of guanosine) or PNPi alone (elevates guanosine in vivo) would be beneficial for the prevention and the treatment of cancer and infections.

### EXAMPLE 2.

*This Example evaluates PNPi as an adjuvant in Tetanus Toxoid Vaccine Efficacy Study.*

Background: Aluminium based mineral salts (Alum) have been used as adjuvants in licensed vaccines for many years. Although alum has been shown to be safe and effective in traditional vaccines where eliciting antibody response is necessary, it is a weak adjuvant for protein subunits, which is one of the major drawbacks. Another limitation of alum is that it fails to induce the *Th1* response associated with the induction of interferon-gamma (interferon-g) and cytotoxic T lymphocytes *(CTL).* Natural control of infectious diseases such as HIV, malaria and tuberculosis that cause the most global mortality are either entirely or partially dependent on the generation of Th1-type immunity. This Example demonstrates that the PNP inhibitors NTR001 (Forodesine set forth structurally herein as Formula II) and NTR002 (Ulodesine set forth structurally herein as Formula I) enhance the potency of the tetanus toxoid vaccine by increasing the antibody titers, and in particular illustrates induction of Th1 responses associated with the induction of interferon-g.

Method: Tetanus toxoid (TT) was used to vaccinate mice thrice, two weeks apart. Mice were treated by oral administration of compounds NTR001 and NTR002 and serum was collected at various time points for antibody titer and interferon-g analysis. Mice in Groups 2-6 (Table 2) are vaccinated subcutaneously with 0.1 ml tetanus toxoid vaccine on DAYS 0, 14 and 28. Mice in Group 1 (Table 1) received no vaccine. Treatments are done as shown in Table 1. Antibody titers for DAYS 38 are determined by ELISA using tetanus toxoid coated microtiter plates and anti-mouse conjugate. Sera from DAY 30 are assayed by ELISA for interferon-g.

**Table 1. Group Compound Treatments**

| **Group** | **No. Mice** | **Test Material** | **ROA** | **Dose (mg/kg)** | **Dose Frequency** |
|---|---|---|---|---|---|
| **1** | **6** | **Vehicle** | **p.o.^{∗}** | **N/A No vaccine** | **Days 0, 14, 28** |
| **2** | **6** | **Vehicle** | **p.o.** | **N/A Vaccinated** | **Days 0, 14, 28** |
| **3** | **6** | **NTR001** | **p.o.** | **30** | **Days 0, 1, 14, 15, 28, 29** |
| **4** | **6** | **NTR001** | **p.o.** | **60** | **Days 0, 14, 28** |
| **5** | **6** | **NTR002** | **p.o.** | **30** | **Days 0, 1, 14, 15, 28, 29** |
| **6** | **6** | **NTR002** | **p.o.** | **60** | **Days 0, 14, 28** |

| | | | | | |
|---|---|---|---|---|---|
| ^{∗}p.o. = oral dose | | | | | |

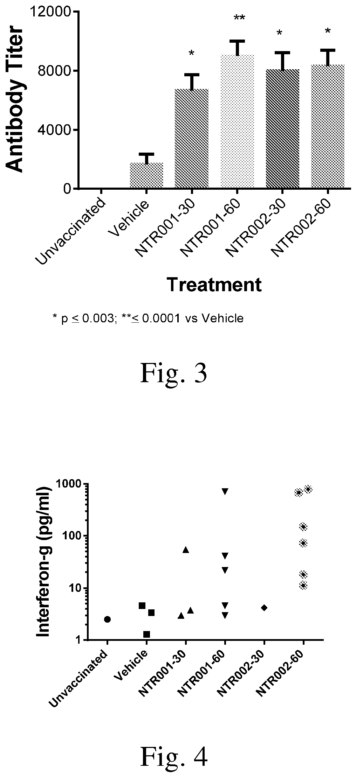

Results: Both NTR001 and NTR002 PNP inhibitors significantly elevated the tetanus toxoid antibody titers compared to the vehicle treated group. The two dosing regimens, 30 mg/kg (given on the day of vaccination and the following day with a total of 6 days of treatment) and 60 mg/kg (given on the day of the vaccination with a total of 3 days of treatment), were effective in increasing the antibody titers (Fig. 3). The interferon-g was elevated in the high dose group (60 mg/kg) for both PNP inhibitors compared to the vehicle treated group (Fig. 4).

Conclusion: PNP inhibitors NTR001 and NTR002 enhance the potency of the tetanus toxoid vaccine by increasing the antibody titers and importantly, the PNP inhibitors induced Th1 responses associated with the induction of interferon-g. Thus, the PNP inhibitors represent a novel approach to enhancing both cellular and humoral immunity and may be useful as a vaccine adjuvant for prevention and treatment of infection and cancer.

### EXAMPLE 3.

*Evaluation of PNPi as anticancer agent in Mouse Melanoma model.*

Chemotherapy is used to treat diverse cancers, but chemotherapy alone is insufficient to cure many advanced cancers, owing to side effects and the limited efficacy against chemo-resistant or relapsing tumors. The need for establishing more efficacious anticancer strategies led to the development of immunotherapies. In this Example PNP inhibitor demonstrate efficacy in reducing tumor volume and/or increasing survival in a syngeneic mouse model of B16 tumors in C57BL/6 mice.

Method: Cancer cells were injected subcutaneously in right flank of each mouse, 1x10⁴ cells in 0.1 ml PBS with 20% Matrigel. Treatment with the NTR001 was initiated on day 6 after injection of tumor cells. Tumor volume and survival were recorded every 3-4 days. Treatment arms were as follows:

**TABLE 2. GROUP TREATMENTS**

| **Group** | **No. Mice** | **Material** | **Dose (mg/kg)** | **ROA** | **Frequency** |
|---|---|---|---|---|---|
| 1 | 10 | Vehicle | 0 | PO | 4 wks (week on/off) |
| 2 | 10 | NTR001 | 30 | PO | 4 wks (week on/off) |
| 3 | 10 | Cyclophosphamide | 100 | IP | Single dose |
| 4 | 10 | Cyclophosphamide NTRPP1 | 100 30 | IP PO | Single dose 4 wks qd/week on/off^{∗} |
| 5 | 10 | NTR001 | 5 | Drinking water | 28 days |
| 6 | 10 | Cyclophosphamide NTR001 | 100 5 | IP Drinking water | Single Dose 28 days |

| | | | | | |
|---|---|---|---|---|---|
| ^{∗}one week on treatment one week off treatment | | | | | |

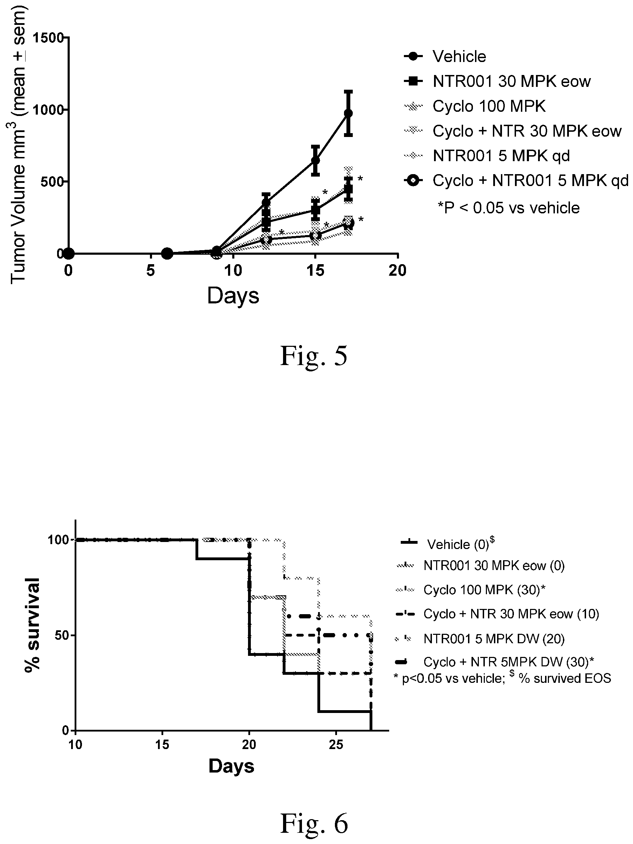

Results: Treatment with NTR001 resulted in a significant decrease in tumor volume (Fig. 5). Treatment with NTR001 demonstrated 0 - 20% survival as single agent (Fig. 6). Cyclcophosphamide and combination of cyclophosphamide with NTR001 at 5 mg/kg dose demonstrated 30% survival whereas there were no survivors in the vehicle treated group.

Conclusion: PNP inhibitor NTR001 demonstrated significant efficacy in the syngeneic mouse melanoma model. Combinations of NTR001 with other anticancer agents is also contemplated. Treatment with alternate doses and dose schedule is also warranted.

### EXAMPLE 4.

*Evaluation of antibacterial activity of PNPi in Mouse Model of L. Monocytogenes Infection.*

In the past, antiviral and antibacterial research has focused mainly on viral and bacterial targets. Due to continued growth of drug resistant organisms the search for effective and differentiated antiviral and antibacterial therapies continues. Development of immune-potentiating agent is one of the strategies being pursued to identify new anti-infective agents. This Example investigates whether PNP inhibitors NTR001 and NTR002 administered by oral and intraperitoneal routes demonstrate antibacterial effect in the mouse model of *Listeria monocytogenes* infection.

Method: Balb/c mice are infected with 1x10⁶ CFU of *L. monocytogenes* (ATCC Strain35152, hemolytic substrain) by intravenous route. The treatment of various groups is initiated -4 hr prior to infection except for Groups 3 and 7 for which treatment was initiated 2 days prior to infection and group 6 and 10 for which treatment was initiated 5 days prior to infection. Weight and survival are the end points of the study. Treatment arms were as follows:

**TABLE 3. TREATMENT GROUPS**

| Group | # mice | Treatment | Dose (mg/kg) | Route | Frequency |
|---|---|---|---|---|---|
| 1 | 5 | Vehicle | 10 ml/kg | PO | DAYS 0, 1, 2 |
| 2 | 10 | Vehicle | " | PO | DAYS 0, 1, 2 |
| 3 | " | NTR001 | 30 | PO | DAYS -2, -1, 0, 1, 2 |
| 4 | " | " | " | PO | DAYS 0, 1, 2 |
| 5 | " | " | " | IP | DAYS 0, 1, 2 |
| 6 | " | " | 2 | DW | DAY-5 thru end |
| 7 | " | NTR002 | 30 | PO | DAYS -2, -1, 0, 1, 2 |
| 8 | " | " | " | PO | DAYS 0, 1, 2 |
| 9 | " | " | " | IP | DAYS 0, 1, 2 |
| 10 | " | " | 2 | DW | DAY-5 thru end |

| | | | | | |
|---|---|---|---|---|---|
| PO = oral gavage; IP = intraperitoneal injection; DW = drinking water | | | | | |

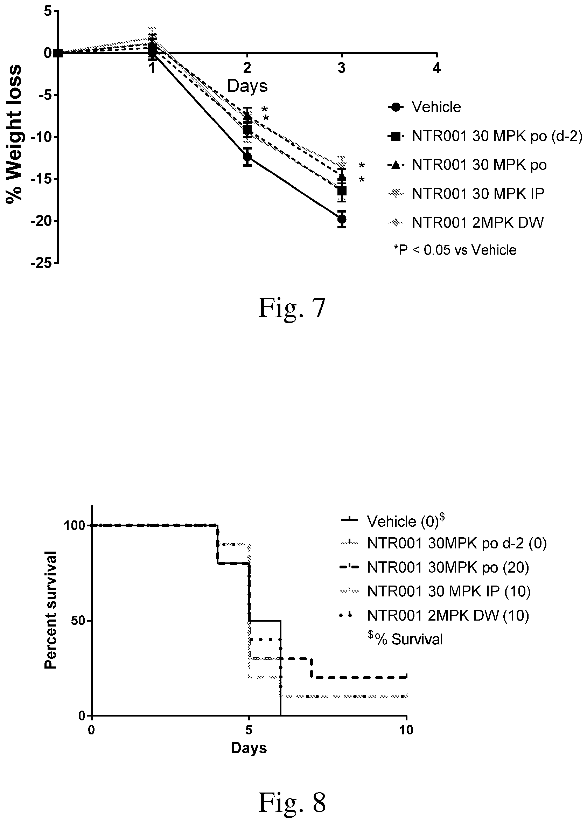

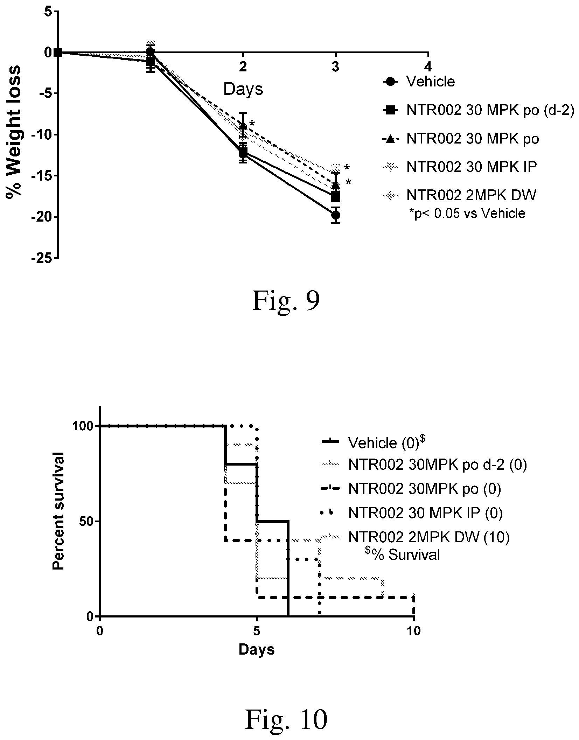

Results: Treatment with NTR001 and NTR002 resulted in significant decrease in weight loss (Figs. 7 and 9) and protection of 10 - 20% of the animals (Figs. 8 and 10).

Conclusion: PNP inhibitors NTR001 and NTR002 demonstrated significant benefit in mouse model of *L. monocytogenes* infection. Combination with other antibacterial agents and treatment with alternate doses and dose schedule is warranted.

### EXAMPLE 5.

*Evaluation of PNPi NTR001 in combination with CTLA4-Ab in Mouse Melanoma model.*

Chemotherapy is used to treat diverse cancers, but chemotherapy alone is insufficient to cure many advanced cancers, owing to side effects and the limited efficacy against chemo-resistant or relapsing tumors. The need for establishing more efficacious anticancer strategies led to the development of immunotherapies. In this example, PNPi in combination with CTLA4-Ab demonstrates efficacy in reducing tumor volume and increasing survival in a syngeneic mouse model of B16 tumors in C57BL/6 mice.

Method: Cancer cells were injected subcutaneously in right flank of each mouse, 1x10⁴ cells in 0.1 ml PBS with 20% Matrigel. Tumor volume and survival were recorded every 2-3 days. Treatment arms were as follows.

| | | **TABLE1: GROUP TREATMENT** | | | |
|---|---|---|---|---|---|
| **Group** | **#Mice** | **Test Material** | **Dose (mg/kg)** | **ROA** | **Frequency** |
| 1 | 10 | Vehicle | 0 | PO* | DAYS 0, 3 and 6 |
| | | Hamster IgG | 100, 50, 50 ug** | IP*** | DAYS 0, 3 and 6 |
| 2 | 10 | NTR001 | 60 | PO | DAYS 0, 3 and 6 |
| | | Hamster IgG | 100, 50, 50 ug | IP | DAYS 0, 3 and 6 |
| 3 | 10 | CTLA Ab | 100, 50, 50 ug | IP | DAYS 0, 3 and 6 |
| 4 | 10 | NTR001 | 60 | PO | DAYS 0, 3 and 6 |
| | | CTLA Ab | 100, 50, 50 ug | IP | DAYS 0, 3 and 6 |

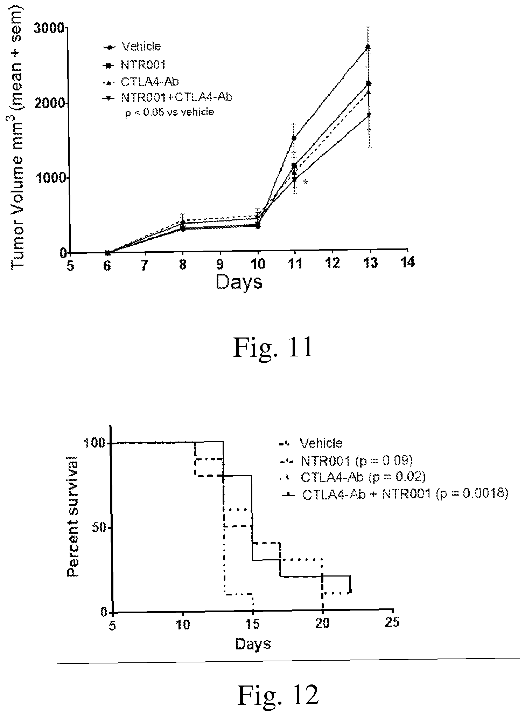

Results: Treatment with NTR001 and CTLA4-Ab (clone 9H10) by itself showed decrease in tumor volume but was not statistically significant. Combination of NTR001 and CTLA4-Ab demonstrated significant decrease in tumor volume (Fig. 11). Combination of NTR001 and CTLA4-Ab and CTLA4-Ab by itself also demonstrated significant improvement in survival (Fig. 12).

Conclusion: Combination of NTR001 and CTLA4-Ab demonstrates significant decrease in tumor volume and significant improvement in survival. Additional dose and dose schedule to be pursued.

## Claims

1. A composition comprising at least one purine nucleoside phosphorylase (PNP) inhibitor for use in a method of enhancing a Graft versus Malignancy effect for the treatment of relapse of malignancy after hematopoietic stem cell transplantation.

2. The composition for use according to claim 1, wherein the method comprises administering a combination of at least one of guanosine, or a pro-drug of guanosine selected from 6-O-methyl guanosine, 6-cyclopropyl amino guanosine, and combinations thereof, or a precursor of guanosine which is guanosine mono phosphate (GMP) or a salt thereof and at least one purine nucleoside phosphorylase (PNP) inhibitor to the patient.

3. A composition for use according to claim 1, wherein the method comprises administering a combination of guanosine and at least one purine nucleoside phosphorylase (PNP) inhibitor to the patient.

4. The composition for use according to claim 2 or 3, wherein the PNP inhibitor is administered simultaneous or subsequent to administering the guanosine.

5. The composition for use according to any of claims 1 to 3 wherein the composition is for administration via an oral or parenteral or topical route.

6. A composition for use according to any of claim 1 to 3, wherein the method comprises administering donor lymphocyte infusion (DLI) to a patient.

7. The composition for use according to any of claims 1 to 3 or 6, wherein the PNP inhibitor is one or more compounds selected from 1,5-dihydro-7-[[(3R,4R)-3-hydroxy-4-(hydroxymethyl) pyrrolidin-1-yl]methyl]-4H-pyrrolo[3,2-d]pyrimidin-4-one, 7-[(2S,3S,4R,5R)-3,4-dihydroxy-5-(hydroxymethyl)-2-pyrrolidinyl]-1,5-dihydro-4H-pyrrolo[3,2-d]pyrimidin-4-one, and 7-[[(2R,3S)-1,3,4-trihydroxybutan-2-ylamino]methyl]-3H-pyrrolo[3,2-d] pyrimidin-4-one.

## Patentansprüche

1. Zusammensetzung, umfassend mindestens einen Inhibitor der Purin-Nukleosid-Phosphorylase (PNP) zur Verwendung in einem Verfahren zum Verstärken einer Graft-versus-Malignität-Wirkung zur Behandlung eines Malignitätsrückfalls nach einer hämatopoetischen Stammzelltransplantation.

2. Zusammensetzung zur Verwendung nach Anspruch 1, wobei das Verfahren Verabreichen einer Kombination aus mindestens einem von Guanosin oder einem Prodrug von Guanosin, ausgewählt aus 6-O-Methylguanosin, 6-Cyclopropylaminoguanosin und Kombinationen daraus, oder einem Präkursor von Guanosin, der Guanosinmonophosphat (GMP) oder ein Salz davon ist, und mindestens einem Inhibitor der Purin-Nukleosid-Phosphorylase (PNP) an den Patienten umfasst.

3. Zusammensetzung zur Verwendung nach Anspruch 1, wobei das Verfahren Verabreichen einer Kombination aus Guanosin und mindestens einem Inhibitor der Purin-Nukleosid-Phosphorylase (PNP) an den Patienten umfasst.

4. Zusammensetzung zur Verwendung nach Anspruch 2 oder 3, wobei der PNP-Inhibitor gleichzeitig mit oder nachfolgend zu dem Verabreichen des Guanosins verabreicht wird.

5. Zusammensetzung zur Verwendung nach einem der Ansprüche 1 bis 3, wobei die Zusammensetzung zur Verabreichung über einen oralen oder parenteralen oder topischen Weg ist.

6. Zusammensetzung zur Verwendung nach einem der Ansprüche 1 bis 3, wobei das Verfahren Verabreichen einer Spenderlymphozyteninfusion (DLI) an einen Patienten umfasst.

7. Zusammensetzung zur Verwendung nach einem der Ansprüche 1 bis 3 oder 6, wobei der PNP-Inhibitor eine oder mehrere Verbindungen, ausgewählt aus 1,5-Dihydro-7-[[(3R,4R)-3-hydroxy-4-(hydroxymethyl)-pyrrolidin-1-yl]methyl]-4H-pyrrolo[3,2-d]pyrimidin-4-on, 7-[(2S,3S,4R,5R)-3,4-dihydroxy-5-(hydroxymethyl)-2-pyrrolidinyl]-1,5-dihydro-4H-pyrrolo[3,2-d]pyrimidin-4-on und 7-[[(2R,3S)-1,3,4-Trihydroxybutan-2-ylamino]methyl]-3H-pyrrolo[3,2-d]-pyrimidin-4-on, ist.

## Revendications

1. Composition comprenant au moins un inhibiteur de purine nucléoside phosphorylase (PNP) pour une utilisation dans un procédé d'amélioration d'un effet greffon versus tumeur maligne pour le traitement d'une récidive de tumeur maligne après transplantation de cellules souches hématopoïétiques.

2. Composition pour une utilisation selon la revendication 1, ledit procédé comprenant l'administration d'une combinaison d'au moins l'un parmi la guanosine, ou un promédicament de guanosine choisi parmi la 6-O-méthylguanosine, la 6-cyclopropylaminoguanosine et leur combinaison, ou un précurseur de la guanosine qui est le monophosphate de guanosine (GMP) ou un sel de celui-ci et d'au moins un inhibiteur de purine nucléoside phosphorylase (PNP) au patient.

3. Composition pour une utilisation selon la revendication 1, ledit procédé comprenant l'administration d'une combinaison de guanosine et d'au moins un inhibiteur de purine nucléoside phosphorylase (PNP) au patient.

4. Composition pour une utilisation selon la revendication 2 ou 3, ledit inhibiteur de PNP étant administré simultanément ou suite à l'administration de la guanosine.

5. Composition pour une utilisation selon l'une quelconque des revendications 1 à 3, ladite composition étant pour une administration via une voie orale ou parentérale ou topique.

6. Composition pour une utilisation selon l'une quelconque des revendications 1 à 3, ledit procédé comprenant l'administration d'une perfusion de lymphocytes de donneur (DLI) à un patient.

7. Composition pour une utilisation selon l'une quelconque des revendications 1 à 3 ou 6, ledit inhibiteur de PNP étant un ou plusieurs composés choisis parmi la 1,5-dihydro-7-[[(3R,4R)-3-hydroxy-4-(hydroxyméthyl)pyrrolidin-1-yl]méthyl]-4H-pyrrolo[3,2-d]pyrimidin-4-one, la 7-[(2S,3S,4R,5R)-3,4-dihydroxy-5-(hydroxyméthyl)-2-pyrrolidinyl]-1,5-dihydro-4H-pyrrolo[3,2-d]pyrimidin-4-one, et la 7-[[(2R,3S)-1,3,4-trihydroxybutan-2-ylamino]méthyl]-3H-pyrrolo[3,2-d]pyrimidin-4-one.
